# EUROPEAN PATENT APPLICATION

(11) **EP 3 620 122 A1**
(43) Date of publication of application: **11.03.2020**
(21) Application number: 18795246.0
(22) Date of filing: 26.01.2018
(51) Int. Cl.: A61B 17/29, A61B 17/94, A61B 34/35, B25J 17/00

(54) **DRIVE UNIT, SURGICAL INSTRUMENT FOR MEDICAL USE, AND OPERATION SYSTEM**

(30) Priority: 01.05.2017 JP 2017091233
(71) Applicant: Medicaroid Corporation, Kobe-shi, Hyogo 650-0047 (JP)
(72) Inventor: NAKANISHI,Tetsuya, Kobe-shi Hyogo 650-0047 (JP); ISHIHARA,Kazuki, Kobe-shi Hyogo 650-0047 (JP)
(74) Representative: Müller Hoffmann & Partner
(86) International application number: PCT/JP2018/002555
(87) International publication number: WO 2018/203425

(57) **Abstract**

A drive unit (101) includes: a first gear (61) that rotates about a first axis (161); a second gear (62) that rotates about a second axis (162) extending in a direction perpendicular to the first axis (161); and a third gear (63) that rotates about a third axis (163) extending in a direction perpendicular to the first axis (161). Elongated elements (13) include: a first elongated element (13a) that is rigid and meshes with the second gear (62); and a second elongated element (13b) that is rigid and meshes with the third gear (63). The second gear (62) and third gear (63) mesh with the first gear (61). The distal end part (11) is operated in such a manner that as the first gear (61) rotates, the first elongated element (13a) moves in a longitudinal direction of the medical treatment tool (4b) while the second elongated element (13b) moves in a direction opposite to the direction in which the first elongated element (13a) moves.

## Description

### TECHNICAL FIELD

The invention relates to a drive unit for operating distal end parts, such as grasping forceps used in surgeries, a medical treatment tool, and a surgical system.

### BACKGROUND ART

In the endoscopic surgery field, surgery assistant robots have been heretofore employed in some cases. A surgery assistant robot includes a patient-side apparatus provided with manipulators, for example. Suitable medical treatment tools are attached to the manipulators and are remotely operated for surgery. As an example of such a medical treatment tool, Patent Literature 1 (Specification of US Patent No. 6394998) discloses a medical treatment tool that uses plural spools as a driving mechanism to wind or unwind flexible elongated elements such as wire for moving the distal end parts, such as grasping forceps.

### CITATION LIST

### PATENT LITERATURE

Patent Literature 1: Specification of US Patent No. 6394998

### SUMMARY OF INVENTION

### PROBLEM TO BE SOLVED BY INVENTION

However, such a surgery assistant robot requires complicated work to assemble medical treatment tools, such as winding flexible members extending from a distal end part to the driving mechanism, around spools, and adjusting the tension of the flexible members while fixing the spools with screws or the like.

The invention was made to solve the aforementioned problem. An object thereof is to provide a drive unit that facilitates assembly of a medical treatment tool, the medical treatment tool, and a surgical system.

### MEANS FOR SOLVING PROBLEM

A drive unit according to an aspect of the invention to achieve the aforementioned object is a drive unit for driving elongated elements to operate a distal end part of a medical treatment tool, the drive unit including: a first gear that rotates about a first axis; a second gear that rotates about a second axis extending in a direction perpendicular to the first axis; and a third gear that rotates about a third axis extending in a direction perpendicular to the first axis. The elongated elements include: a first elongated element that is rigid and meshes with the second gear; and a second elongated element that is rigid and meshes with the third gear, and the second and third gears mesh with the first gear. The distal end part is operated in such a manner that as the first gear rotates, the first elongated element moves in a longitudinal direction of the medical treatment tool while the second elongated element moves in a direction opposite to the direction in which the first elongated element moves.

A medical treatment tool according to an aspect of the invention to achieve the aforementioned object includes: the drive unit; the distal end part that is operated by the first and second elongated elements; and a shaft that accommodates the first and second elongated elements.

A medical treatment tool according to another aspect of the invention to achieve the aforementioned object includes: a driving mechanism that includes three of the drive units; the distal end part that is controlled with three pairs of the first and second elongated elements; and a shaft that accommodates the three pairs of the first and second elongated elements.

A surgical system according to an aspect of the invention to achieve the aforementioned object includes: the medical treatment tool; and a manipulator including the transmitter member that engages with the receiver member.

### EFFECT OF INVENTION

According to the invention, it is possible to further facilitate assembly of a medical treatment tool.

### BRIEF DESCRIPTION OF DRAWINGS

Fig. 1 is a view illustrating a configuration of a surgical system;
Fig. 2 is a view illustrating a configuration of a medical treatment tool;
Fig. 3 is a perspective view illustrating a configuration of a distal end part of the medical treatment tool illustrated in Fig. 2;
Fig. 4 is a side view illustrating the configuration of the distal end part of the medical treatment tool illustrated in Fig. 3;
Fig. 5 is a view illustrating a configuration of a combination of rods and a wire, as an example of an elongated element used in the medical treatment tool;
Fig. 6 is a view illustrating an arrangement example of wound wires;
Fig. 7 is a view illustrating a configuration of a distal end part in a modification of a medical treatment tool;
Fig. 8 is a perspective view illustrating a configuration of a driving mechanism and a driving device;
Fig. 9 is a front view illustrating the configuration of the driving mechanism illustrated in Fig. 8;
Fig. 10 is a perspective view illustrating a configuration example of a transmission section;
Fig. 11 is a cross-sectional view illustrating a configuration of a medical treatment tool (Example 1);
Fig. 12 is a cross-sectional view illustrating a configuration of a medical treatment tool (Example 2); and
Fig. 13 is a perspective view illustrating a configuration of a medical treatment tool (Example 3).

### EMBODIMENTS FOR CARRYING OUT THE INVENTION

### (Surgical System)

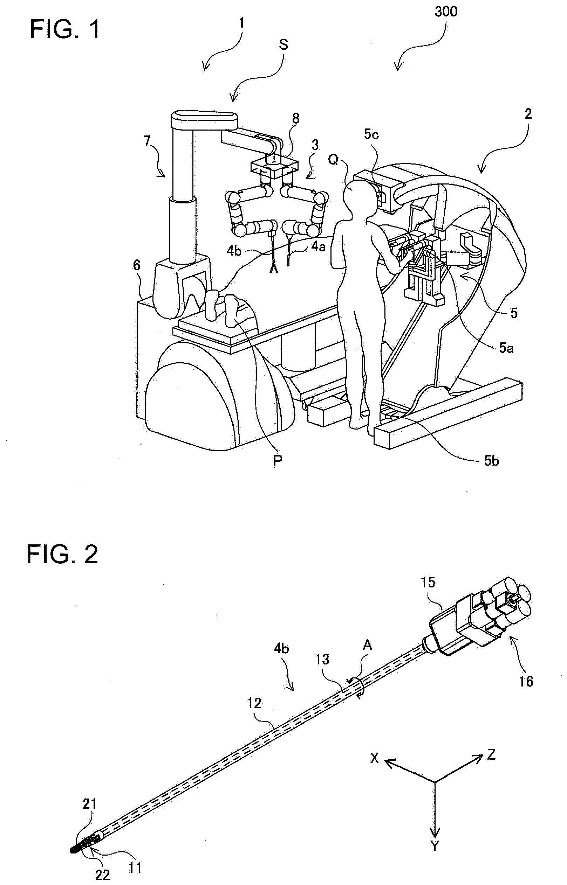

Fig. 1 is a view illustrating a configuration of a surgical system.

With reference to Fig. 1, a surgical system 300 is, for example, a system in which a surgeon Q uses a patient-side apparatus 1 to perform an endoscopic surgery for a subject P, including a human or an animal. The surgical system 300 includes the patient-side apparatus 1 and an operation apparatus 2 for operating the patient-side apparatus 1.

The surgeon Q inputs a movement instruction for the patient-side apparatus 1 into the operation apparatus 2, and the operation apparatus 2 transmits an instruction signal including the inputted movement instruction, to the patient-side apparatus 1. The patient-side apparatus 1 receives the instruction signal transmitted from the operation apparatus 2 and operates an endoscopic assembly 4a and a medical treatment tool 4b, which are connected to distal ends of the patient-side apparatus 1, based on the movement instruction included in the received instruction signal.

To be more specific, the operation apparatus 2 includes an operation input section 5, which includes an operation manipulator 5a and an operation pedal 5b, and a monitor 5c. The monitor 5c displays images captured by the endoscopic assembly 4a. The operation manipulator 5a and operation pedal 5b are equipment for the surgeon Q to input a movement instruction.

The surgeon Q inputs a movement instruction to the operation apparatus 2 by operating the operation manipulator 5a and operation pedal 5b while watching the diseased area in the image displayed on the monitor 5c. The operation apparatus 2 transmits an instruction signal including the inputted movement instruction, to the patient-side apparatus 1 via wire or wirelessly.

The patient-side apparatus 1 includes: a positioner 7; a platform 8, which is attached to the top end of the positioner 7; manipulators 3, which are detachably attached to the platform 8; the endoscopic assembly 4a; the medical treatment tool 4b; and a controller 6 that controls movement of the patient-side apparatus 1.

The endoscopic assembly 4a and medical treatment tool 4b are attached to the respective manipulators 3. The medical treatment tool 4b is grasper forceps (a grasper), a needle driver, scissors, or the like.

The controller 6 receives an instruction signal from the operation apparatus 2 and operates the endoscopic assembly 4a and medical treatment tool 4b based on the received instruction signal.

Specifically, the controller 6 receives an instruction signal and based on a movement instruction included in the instruction signal, first controls the positioner 7 for positioning of the platform 8. The controller 6 also positions the manipulators 3 so that the endoscopic assembly 4a and medical treatment tool 4b are located at predetermined initial positions relative to a not-illustrated cannula placed on the body surface of the subject P.

Based on the movement instruction, the controller 6 then outputs a control signal to drive the endoscopic assembly 4a or medical treatment tool 4b, to the endoscopic assembly 4a or medical treatment tool 4b via the manipulators 3. The endoscopic assembly 4a and medical treatment tool 4b then operate in accordance with the control signals received from the controller 6.

### (Medical treatment tool)

### (Entire Configuration)

Fig. 2 is a view illustrating the configuration of the medical treatment tool.

With reference to Fig. 2, the medical treatment tool 4b includes a distal end part 11, a shaft 12, elongated elements 13 to operate the distal end part 11, and a driving mechanism 15 to drive the elongated elements 13. The driving mechanism 15 is activated by a driving device 16 including a driving source. The driving device 16 is a part of the corresponding manipulator 3 and is provided at the distal end of the manipulator 3, for example.

In this specification, the term "elongated elements" collectively means rods, wires, cables, or the like. Rigid ones, such as rods, are referred to as rigid elongated elements while flexible ones, such as wire and cable, are referred to as flexible elongated elements. In order to describe elongated elements without distinguishing rigid and flexible ones, the elongated elements, including rigid and flexible ones, are collectively referred to as elongated elements.

The distal end part 11 includes two jaws 21 and 22, for example. These two jaws 21 and 22 have the same shape to minimize the manufacturing cost. The shaft 12 includes a cylindrical shape extending in the longitudinal direction of the medical treatment tool 4b and is rotatable in the direction of arrow A. The shaft 12 is thus rotatable about the axis along the longitudinal direction thereof.

As the elongated elements 13, rods, for example, are rigid members accommodated in the shaft 12. As the elongated elements 13, wires, for example, are flexible members which are made of tungsten, stainless, or the like and have sufficient strength, flexibility, and durability.

The driving mechanism 15 detachably connects to the corresponding manipulator 3 in the patient-side apparatus 1 illustrated in Fig. 1. The driving mechanism 15 receives a control signal from the patient-side apparatus 1 via the manipulator 3 and moves the elongated elements 13 in the longitudinal direction of the medical treatment tool 4b in accordance with the control signal. The detailed configuration of the driving mechanism 15 is described later.

### (Distal end part)

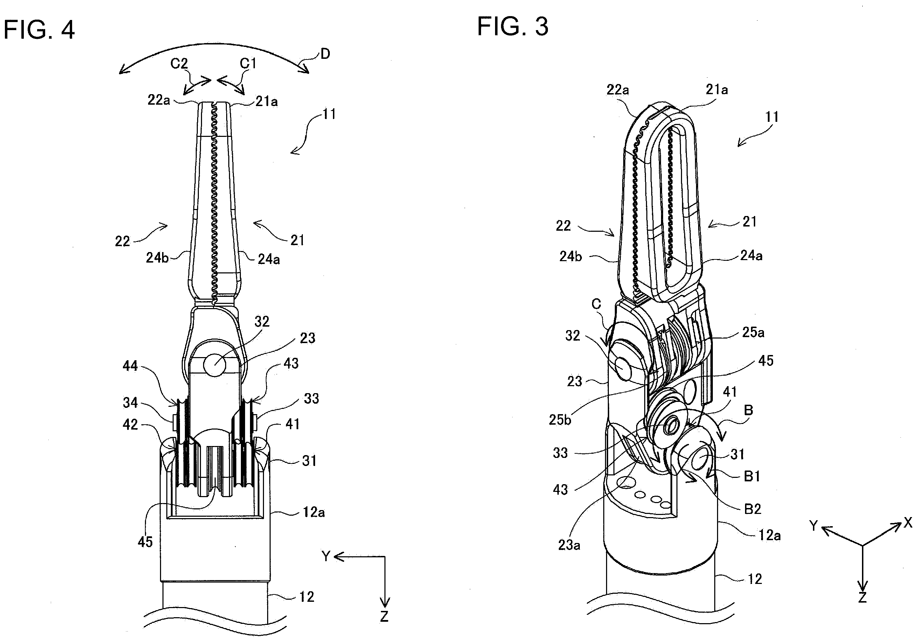

Fig. 3 is a perspective view illustrating the configuration of the distal end part of the medical treatment tool illustrated in Fig. 2.

With reference to Fig. 3, the distal end part 11 includes a wrist section 23, in addition to the jaws 21 and 22. The wrist section 23 is attached to an end section 12a of the shaft 12 through a joint section 31. The wrist section 23 is pivotable about the joint section 31 in the direction of arrow B.

The jaws 21 and 22 are attached to the wrist section 23 through a joint section 32. The jaws 21 and 22 include finger sections 24a and 24b and pulley sections 25a and 25b, respectively. The finger sections 24a and 24b are pivotable about the joint section 32 in the direction of arrow C. The pulley sections 25a and 25b are rotatable about the joint section 32.

The direction that the joint section 31 extends is preferably different from the direction that the joint section 32 extends. Herein, it is assumed that the direction that the joint section 31 extends is at 90 degrees to the direction that the joint section 32 extends. Hereinafter, the direction that the joint section 31 extends is the direction of Y axis; the direction that the joint section 32 extends is the direction of X axis; and the longitudinal direction of the shaft 12 is the direction of Z axis.

Fig. 4 is a side view illustrating the configuration of the distal end part of the medical treatment tool illustrated in Fig. 3.

With reference to Fig. 4, free ends 21a and 22a of the jaws 21 and 22 pivot about the joint section 32 as indicated by arrows C1 and C2, respectively. The free ends 21a and 22a thereby move close to and away from each other or pivot in the same direction.

The distal end part 11 further includes a first pulley section 41, a second pulley section 42, a third pulley section 43, a fourth pulley section 44, and a fifth pulley section 45, in addition to the jaws 21 and 22 and wrist section 23. Each of the first to fourth pulley sections 41 to 44 includes an inner pulley and an outer pulley.

The first, second, and fifth pulley sections 41, 42, and 45 are attached to the end section 12a through the joint section 31 and are rotatable about the joint section 31. The third pulley section 43 is attached to the wrist section 23 through a joint section 33 and is rotatable about the joint section 33. The fourth pulley section 44 is attached to the wrist section 23 through a joint section 34 and is rotatable about the joint section 34.

The planes of rotation of the first pulley section 41 and third pulley section 43 lie in the substantially same plane. The planes of rotation of the second pulley section 42 and fourth pulley section 44 lie in the substantially same plane.

### (Rod and Wire)

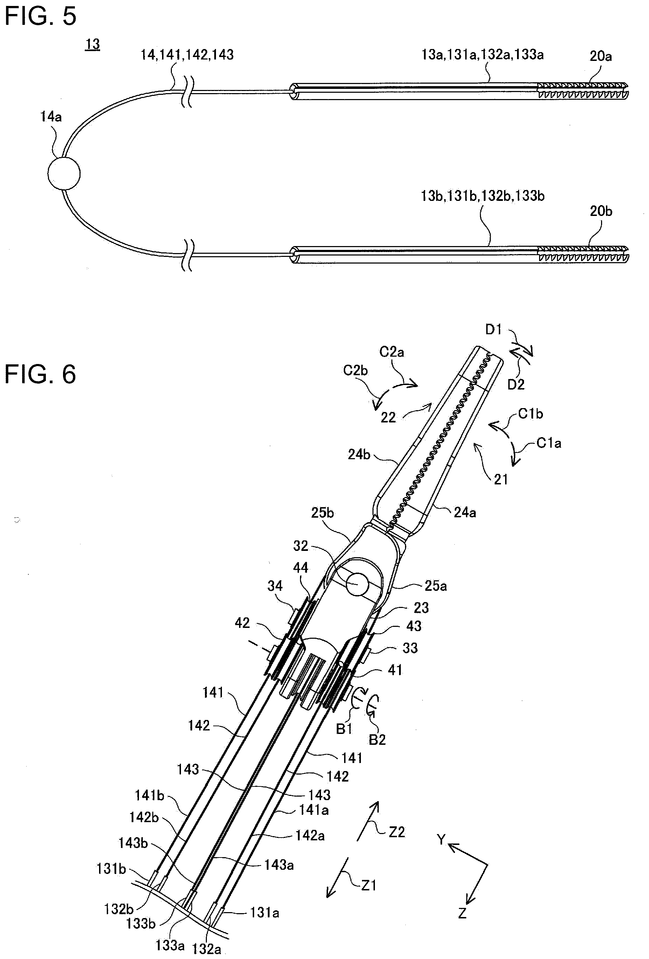

Fig. 5 is a view illustrating the configuration of a combination of rods and a wire as an example of the elongated element used in the medical treatment tool.

With reference to Fig. 5, each elongated element 13 includes a first rod (a first rigid elongated element) 13a, a second rod (a second rigid elongated element) 13b, and a wire 14. The first and second rods 13a and 13b individually connect to the wire 14. The wire 14 is provided with a thick section 14a near the center thereof.

Herein, the first and second rods 13a and 13b connect to the same wire 14. However, the wire 14 connected to the first rod 13a may be a different member from the wire 14 connected to the second rod 13b.

The first and second rods 13a and 13b include toothed sections 20a and 20b at ends on the opposite side to the wire 14. The toothed sections 20a and 20b mesh with a gear of the driving mechanism 15.

The medical treatment tool 4b illustrated in Fig. 2 includes three sets of the first rod 13a, second rod 13b, and wire 14, for example. Hereinafter, the three first rods 13a included in the three sets are referred to as first rods 131a, 132a, and 133a. The three second rods 13b included in the three sets are referred to as second rods 131b, 132b, and 133b. The three wires 14 included in the three sets are referred to as wires 141, 142, and 143.

The wire 141 connects the first and second rods 131a and 131b. The wire 142 connects the first and second rods 132a and 132b. The wire 143 connects the first and second rods 133a and 133b.

Fig. 6 is a view illustrating an example of wound wires. In Fig. 6, arrow Z1 indicates the positive direction of the Z axis while arrow Z2 indicates the negative direction of the Z axis.

With reference to Fig. 6, in the assembly process of the medical treatment tool 4b, the wire 141 is wound around the outer pulley of the first pulley section 41 and the outer pulley of the third pulley section 43, then wound around the pulley section 25b of the jaw 22, and then wound around the inner pulley of the fourth pulley section 44 and the inner pulley of the second pulley section 42.

The thick section 14a of the wire 141 illustrated in Fig. 5 is fitted to a not-illustrated recess formed in the finger section 24b of the jaw 22 illustrated in Fig. 6, for example. The wire 141 is thereby fixed to the jaw 22, so that the jaw 22 moves through the wire 141 being driven.

The wire 142 is wound around the inner pulley of the first pulley section 41 and the inner pulley of the third pulley section 43, wound around the pulley section 25a of the jaw 22, and then wound around the outer pulley of the fourth pulley section 44 and the outer pulley of the second pulley section 42.

The thick section 14a of the wire 142 illustrated in Fig. 5 is fitted to a not-illustrated recess formed in the finger section 24a of the jaw 21 illustrated in Fig. 6, for example. The wire 142 is thereby fixed to the jaw 21, so that the jaw 21 moves through the wire 142 being driven.

The wire 143 is wound around the fifth pulley section 45. The thick section 14a of the wire 143 illustrated in Fig. 5 is fitted to a not-illustrated recess formed in the wrist section 23 illustrated in Fig. 6, for example. The wire 143 is thereby fixed to the wrist section 23, so that the wrist section 23 moves through the wire 143 being driven.

In such a manner, the jaws 22 and 21 and wrist section 23 of the distal end part 11 move through the wires 141, 142, and 143 as the flexible member being driven, respectively, so that the distal end part 11 operates smoothly in a desired direction.

### (Movement of Distal end part)

When the first rod 131a is pulled in the direction of arrow Z1, the jaw 22 pivots about the joint section 32 in the direction of arrow C2a. In other words, the jaw 22 pivots towards the jaw 21 in the circumferential direction of a circle centered at the joint section 32. When the second rod 131b is pulled in the direction of arrow Z1, the jaw 22 pivots about the joint section 32 in the direction of arrow C2b. In other words, the jaw 22 pivots away from the jaw 21 in the circumferential direction of a circle centered at the joint section 32.

When the first rod 132a is pulled in the direction of arrow Z1, the jaw 21 pivots about the joint section 32 in the direction of arrow C1a. In other words, the jaw 21 pivots away from the jaw 22 in the circumferential direction of a circle centered at the joint section 32. When the second rod 132b is pulled in the direction of arrow Z1, the jaw 21 pivots about the joint section 32 in the direction of arrow C1b. In other words, the jaw 21 pivots toward the jaw 22 in the circumferential direction of a circle centered at the joint section 32.

When the second rod 131b and first rod 132a are pulled in the direction of arrow Z1 simultaneously, the jaws 21 and 22 pivot away from each other in the circumferential direction of a circle centered at the joint section 32. When the first rod 131a and second rod 132b are pulled in the direction of arrow Z1 simultaneously, the jaws 21 and 22 pivot toward each other in the circumferential direction of a circle centered at the joint section 32.

When the first rods 131a and 132a are pulled in the direction of arrow Z1 simultaneously, the jaws 21 and 22 both pivot in the circumferential direction of a circle centered at the joint section 32, as indicated by arrow D1. In other words, the jaw 21 pivots in the direction of arrow C1a while the jaw 22 pivots in the direction of arrow C2a.

When the second rods 131b and 132b are pulled in the direction of arrow Z1 simultaneously, the jaws 21 and 22 both pivot in the circumferential direction of a circle centered at the joint section 32, as indicated by arrow D2. In other words, the jaw 21 pivots in the direction of arrow C1b while the jaw 22 pivots in the direction of arrow C2b.

When the first rod 133a is pulled in the direction of arrow Z1, the wrist section 23, which is illustrated in Figs. 3 and 4, pivots about the joint section 31 in the direction of arrow B2. In other words, the wrist section 23 pivots counterclockwise in the circumferential direction of a circle centered at the joint section 31, as seen along the Y axis from the negative side toward the positive side. When the second rod 133b is pulled in the direction of arrow Z1, the wrist section 23 pivots about the joint section 31 in the direction of arrow B1. In other words, the wrist section 23 pivots clockwise in the circumferential direction of a circle centered at the joint section 31, as seen along the Y axis from the negative side toward the positive side.

In such a manner, the jaw 21, jaw 22, and wrist section 23, which are fixed to the wires 141, 142, and 143, respectively, are independently driven through the first rods 131a, 132a, and 133a and the second rods 131b, 132b, and 133b being driven.

### (Modification of Medical treatment tool)

The distal end part 11 of the medical treatment tool 4b is not limited to the configuration in which the distal end part 11 is operated by the elongated elements 13 each including a combination of rods and wire and may have a configuration in which the distal end part 11 is operated by elongated elements each including only rods.

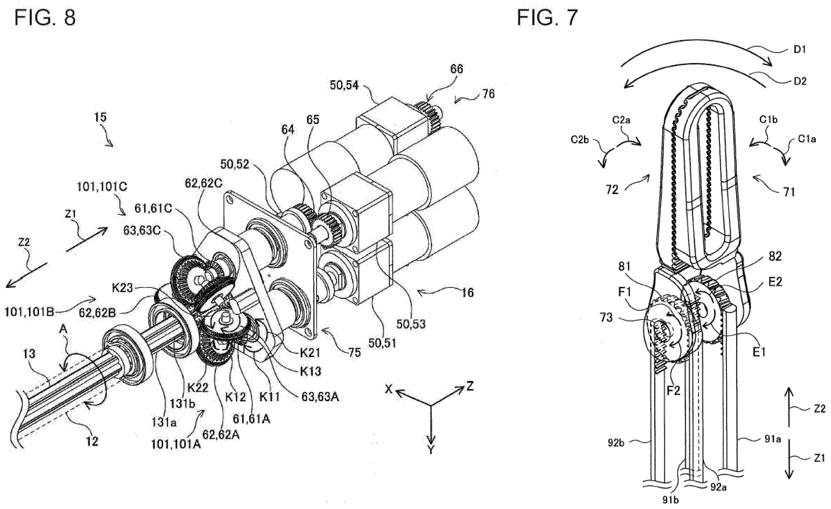

Fig. 7 is a view illustrating the configuration of a distal end part of a modification of a medical treatment tool.

With reference to Fig. 7, the distal end part 11 includes two jaws 71 and 72 and two gears 81 and 82, for example. The two jaws 71 and 72 and two gears 81 and 82 are attached to the end section 12a of the shaft 12 illustrated in Fig. 3, through a joint section 73. The gears 81 and 82 are rotatable about the joint section 73, and the jaws 71 and 72 are pivotable about the joint section 73 in the direction of arrow D1 or D2.

The medical treatment tool 4b includes first rods (first rigid elongated elements) 91a and 92a and second rods (second rigid elongated elements) 91b and 92b instead of the rods and wires.

The first and second rods 91a and 91b are provided so that teeth formed at respective ends face each other on either side of the gear 81. As the first and second rods 91a and 91b move in opposing directions along the Z axis, the gear 81 rotates about the joint section 73. The jaw 71 thereby pivots about the joint section 73 together with the gear 81.

The first and second rods 92a and 92b are provided so that teeth formed at respective ends face each other on either side of the gear 82. As the first and second rods 92a and 92b move in opposing directions along the Z axis, the gear 82 rotates about the joint section 73. The jaw 72 thereby pivots about the joint section 73 together with the gear 82.

Specifically, when the first rod 91a is pulled in the direction of arrow Z1, the second rod 91b moves in the direction of arrow Z2, and the gear 81 rotates in the direction of arrow E1. The jaw 71 thereby pivots in the direction of arrow C1a. When the first rod 91a is pulled in the direction of arrow Z2, the second rod 91b moves in the direction of arrow Z1, and the gear 81 rotates in the direction of arrow E2. The jaw 71 thereby pivots in the direction of arrow C1b.

When the first rod 92a is pulled in the direction of arrow Z1, the second rod 92b moves in the direction of arrow Z2, and the gear 82 rotates in the direction of arrow F1. The jaw 72 thereby pivots in the direction of arrow C2a. When the first rod 92a is pulled in the direction of arrow Z2, the second rod 92b moves in the direction of arrow Z1, and the gear 82 rotates in the direction of arrow F2. The jaw 72 thereby pivots in the direction of arrow C2b.

When the first rods 91a and 92a and second rods 91b and 92b directly connect to the distal end part 11 in such a manner, no work is necessary to adjust wire tension during the assembly process of the medical treatment tool 4b or other processes. It is therefore possible to further facilitate assembly of the medical treatment tool 4b and the like.

### (Driving Mechanism and Driving Device)

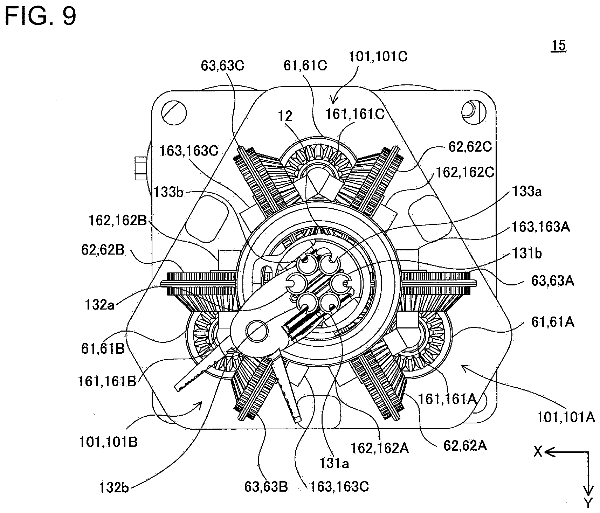

Fig. 8 is a perspective view illustrating the configurations of the driving mechanism and driving device. Fig. 9 is a front view illustrating the configuration of the driving mechanism illustrated in Fig. 8. Figs. 8 and 9 do not illustrate a housing that covers the driving mechanism 15 for explanation of the driving mechanism 15.

With reference to Fig. 8, the driving mechanism 15 includes plural drive units 101. Each drive unit 101 includes a first gear 61; a second gear 62, which meshes with the first rod 13a; a third gear 63, which meshes with the second rod 13b; and a receiver member as a part of a transmission section 75 to rotate the first gear 61. The transmission section 75 is described in detail later.

The driving device 16 includes plural actuators 50. Each actuator 50 receives a control signal from the patient-side apparatus 1 illustrated in Fig. 1, through the manipulator 3 and rotates the corresponding receiver member of the transmission section 75 in accordance with the control signal. The receiver member rotates itself to rotate the corresponding first gear 61.

To be more specific, with reference to Fig. 9, the second and third gears 62 and 63 have substantially the same shape. The first gear 61 rotates about a first axis 161 parallel to the longitudinal direction of the medical treatment tool 4b. The second gear 62 rotates about a second axis 162, which extends perpendicularly to the first axis 161. The third gear 63 rotates about a third axis 163, which extends perpendicularly to the first axis 161.

The first gear 61 is a substantially-conical bevel gear, for example. The first gear 61 includes teeth in the conical surface. Each of the second and third gears 62 and 63 is a gear including a combination of a substantially-conical bevel gear and a cylindrical gear, for example. In the second and third gears 62 and 63, teeth are formed in the lateral surface of the conical part, and teeth are formed in the side surface of the cylindrical part.

The second and third gears 62 and 63 mesh with the first gear 61. To be more specific, the teeth in the lateral surface of the second gear 62 and the teeth in the lateral surface of the third gear 63 mesh with the teeth in the first gear 61.

As the first gear 61 rotates, the second and third gears 62 and 63 rotate in the same direction as seen from the first gear 61 side. Specifically, when the second gear 62 rotates clockwise as seen from the first gear 61 side, the third gear 63 also rotates clockwise as seen from the first gear 61.

In the aforementioned example, the first, second, and third gears 61, 62, and 63 are all bevel gears. However, the first gear 61 may be a crown gear while the second and third gears 62 and 63 are spur gears. Such a configuration also implements the same rotary movement as described above.

The teeth formed in the side surface of the cylindrical part of the second gear 62 mesh with the toothed section 20a formed in the first rod 131a illustrated in Fig. 5. The second gear 62 and first rod 131a constitute a rack-and-pinion structure. The first rod 131a moves in the longitudinal direction of the shaft 12 as the second gear 62 rotates.

The teeth formed in the side surface of the cylindrical part of the third gear 63 mesh with the toothed section 20b, which is formed in the second rod 131b illustrated in Fig. 5. The third gear 63 and second rod 131b constitute a rack-and-pinion structure. The second rod 131b moves in the longitudinal direction of the shaft 12 as the third gear 63 rotates.

The first and second rods 131a and 131b are positioned near the center of the shaft 12. To be more specific, the first rod 131a and second rod 131b are positioned so that the position of engagement of the first rod 131a in the side surface of the disk of the second gear 62 as seen from the first gear 61 is opposite to the position of engagement of the second rod 131b in the side surface of the disk of the third gear 63 as seen from the first gear 61. Specifically, the second gear 62 meshes with the first rod 131a on the right side as seen from the first gear 61 while the third gear 63 meshes with the second rod 131b on the left side as seen from the first gear 61.

The first and second rods 131a and 131b thereby move in opposing directions along the longitudinal axis of the shaft 12 as the first gear 61 rotates.

Such a configuration that the distal end part 11 is operated by using the first and second rods 131a and 131b that move in opposing directions as the first gear 61 rotates eliminates the need for work to wind the wire 14 around the driving mechanism 15 and adjust the tension thereof. It is therefore possible to further facilitate assembly of the medical treatment tool 4b. In addition, in the configuration using the first and second rods 131a and 131b, which are made of a rigid material, the elongated elements do not stretch even after long-term use, unlike flexible members such as wire, thus ensuring strength and durability.

The driving mechanism 15 includes three drive units 101, each including the aforementioned first, second, and third gears 61, 62, and 63, for example. Hereinafter, the three drive units 101 are referred to as drive units 101A, 101B, and 101C. The drive units 101 A, 101B, and 101C are positioned at substantially equal intervals in the circumferential direction of the shaft 12. The rotational axes of the first gears 61 of the drive units 101 are positioned at substantially equal intervals in particular.

The first, second, and third gears 61, 62, and 63 included in the drive unit 101A are referred to as first, second, and third gears 61A, 62A, and 63A, respectively. The first, second, and third gears 61, 62, and 63 included in the drive unit 101B are referred to as first, second, and third gears 61B, 62B, and 63B, respectively. The first, second, and third gears 61, 62, and 63 included in the drive unit 101C are referred to as first, second, and third gears 61C, 62C, and 63C, respectively.

In the drive unit 101A, the second and third gears 62A and 63A mesh with the first gear 61A. The second gear 62A meshes with the first rod 131a to operate the jaw 22 while the third gear 63A meshes with the second rod 131b to operate the jaw 22.

In the drive unit 101B, the second and third gears 62B and 63B mesh with the first gear 61B. The second gear 62B meshes with the first rod 132a to operate the jaw 21 while the third gear 63B meshes with the second rod 132b to control the jaw 21.

In the drive unit 101C, the second and third gears 62C and 63C mesh with the first gear 61C. The second gear 62C meshes with the first rod 133a to operate the wrist section 23 while the third gear 63C meshes with the second rod 133b to operate the wrist section 23.

With reference to Fig. 8 again, the driving device 16 includes four actuators 51, 52, 53, and 54 as the plural actuators 50, for example. The actuators 51, 52, 53, and 54 are servo motors, for example.

The driving device 16 further includes a fourth gear 64, a fifth gear 65, a rotation mechanism 76, and a transmitter member 78 as a part of the transmission section 75. The fourth gear 64 rotates the first gear 61C through the transmission section 75. The fifth gear 65 meshes with the fourth gear 64.

The actuator 51 rotates the first gear 61A through the transmission section 75 in accordance with a control signal from the patient-side apparatus 1 (illustrated in Fig. 1). The actuator 52 rotates the first gear 61B through the transmission section 75 in accordance with a control signal from the patient-side apparatus 1 in a similar manner to the actuator 51.

The actuator 53 rotates the fifth gear 65 in accordance with a control signal from the patient-side apparatus 1. As the fifth gear 65 rotates, the fourth gear 64 rotates to rotate the first gear 61C through the transmission section 75.

The actuator (a rotation mechanism driving actuator) 54 rotates a sixth gear 66, which constitutes the rotation mechanism 76, in accordance with a control signal from the patient-side apparatus 1. As the sixth gear 66 rotates, the whole medical treatment tool 4b rotates about an axis parallel to the longitudinal axis (a shaft axis) of the shaft 12.

Specifically, the rotation mechanism 76 includes a fixed rotational axis adjustment gear (a sun gear, not illustrated) having a disk shape, in addition to the sixth gear 66. The side surface of the disk of the rotational axis adjustment gear meshes with the sixth gear 66. The central axis of the rotational axis adjustment gear coincides with the shaft axis. The sixth gear 66 rotates about its own axis while revolving around the rotational axis adjustment gear (functioning as a planet gear), so that the distal end part 11 of the medical treatment tool 4b rotates about the shaft axis.

The aforementioned configuration that the actuator 54 rotates the shaft 12 in the direction of arrow A allows the direction of the distal end part 11 to change appropriately.

The rotation mechanism 76 needs to have a configuration that allows the medical treatment tool 4b to rotate about an axis parallel to the longitudinal direction (the shaft axis) of the shaft 12. The rotation mechanism 76 unnecessarily employs such a planetary gear mechanism.

### (Transmission Section)

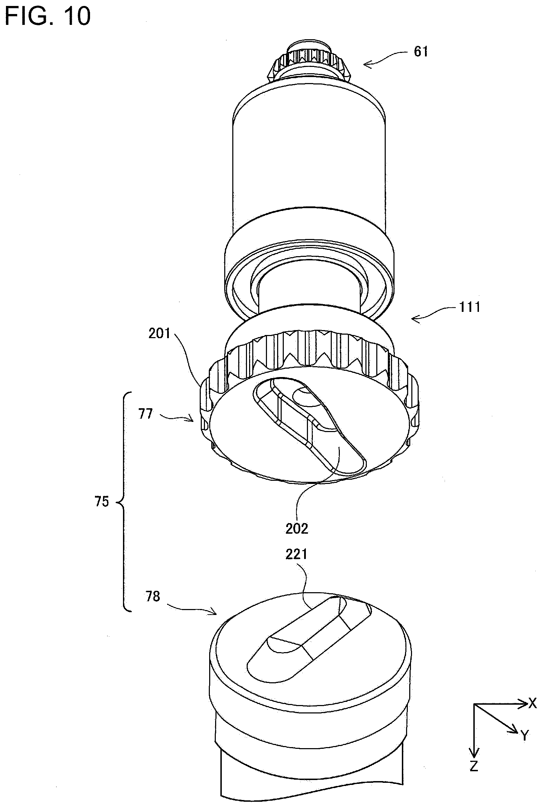

Fig. 10 is a perspective view illustrating the configuration of the transmission section.

With reference to Fig. 10, the transmission section 75 includes a receiver member 77 of the driving mechanism 15; and a transmitter member 78 of the driving device 16.

The receiver member 77 connects to the first gear 61 through a rotation section 111. The receiver member 77 engages with the transmitter member 78 and rotates when the transmitter member 78 rotates. The transmitter member 78 is rotationally driven directly or indirectly by the corresponding actuator 50 illustrated Fig. 8.

To be more specific, the receiver member 77 includes a rotatable disk section 201. In the main surface of the disk section 201, an engaged section 202 is formed. The transmitter member 78 includes an engaging section 221, which is engageable with the engaged section 202 of the receiver member 77.

The engaged section 202 of the receiver member 77 is a groove or a recess, for example. In the main surface of the disk section 201, the engaged section 202 has a continuous pattern which is not symmetric about any line through the center of the main surface. The engaging section 221 of the transmitter member 78 is a protrusion, for example. In the main surface of the transmitter member 220, the engaging section 221 has a continuous pattern which is not symmetric about any line through the center of the main surface, similarly to the engaged section 202.

With such a configuration, the direction of the receiver member 77 to drive the distal end part 11 illustrated in Fig. 2 is determined uniquely among 360 degrees.

The patterns of the engaging section 221 and engaged section 202 are linear, for example. In addition, the pattern of the engaging section 221 passes through the center of the main surface of the transmitter member 78 while the pattern of the engaged section 202 passes through the center of the main surface of the disk section 201.

The engaging section 221 is tapered so as to gradually decrease in width toward the receiver member 77 in the direction of the Z axis, which is perpendicular to the main surface of the transmitter member 78, that is, in the negative direction of the Z axis illustrated in Fig. 10.

The transmitter member 78 is energized toward the receiver member 77, that is, in the negative direction of the Z axis illustrated in Fig. 10, by a spring or the like. Some misalignment in the positional relationship between the engaged section 202 and the engaging section 221 is corrected during assembly thereof so that the engaging section 221 fits into the engaged section 202. This further facilitates the work to engage the engaging section 221 and the engaged section 202.

The engaging section 221 may be a groove or a recess instead of a protrusion. When the engaging section 221 is a groove or a recess, the engaged section 202 is a protrusion which is engageable with the engaging section 221.

The shape of the engaged section 202 needs to be formed in a continuous pattern which is not symmetric about any line through the center of the disk section 201 in the rotation surface of the disk section 201. The shape of the engaged section 202 is not limited to the aforementioned shape.

The receiver member 77 does not need to directly engage with the transmitter member 78. The movement of the transmitter member 78 may be transmitted to the receiver member 77 through an adaptor integrated with a drape.

### (Movement of Distal end part with Rotation of First Gear)

With reference to Fig. 8 again, the actuators 51, 52, and 53 rotate the first gears 61A, 61B, and 61C in accordance with a control signal, respectively, as described above.

When the first gear 61A rotates, the second and third gears 62A and 63A rotate, and the first rod 131a, which meshes with the second gear 62A, and the second rod 121b, which meshes with the third gear 63A, move in opposing directions along the Z axis. When the first rod 131a or second rod 131b is pulled in the direction of arrow Z1, thus, the jaw 22 pivots in the direction of arrow C1a or C1b illustrated in Fig. 6.

Specifically, when the first gear 61A rotates in the direction of arrow K11 illustrated in Fig. 8, the second gear 62A rotates in the direction of arrow K12, and the first rod 131a, which meshes with the second gear 62A, moves in the direction of arrow Z2. In this case, the third gear 63A rotates in the direction of arrow K13, and the second rod 131b, which connects to the third gear 63A, moves in the direction of arrow Z1.

When the first rod 131a moves in the direction of arrow Z2 while the second rod 131b moves in the direction of arrow Z1 in such a manner, the jaw 22 pivots in the direction of arrow C2b as illustrated in Fig. 6.

When the first gear 61A rotates in the direction of arrow K21, the second gear 62A rotates in the direction of arrow K22, and the first rod 131a, which connects to the second gear 62A, moves in the direction of arrow Z1. In this case, the third gear 63A rotates in the direction of arrow K23, and the second rod 131b, which connects to the third gear 63A, moves in the direction of arrow Z2.

When the first rod 131a moves in the direction of arrow Z1 while the second rod 131b moves in the direction of arrow Z2 in such a manner, the jaw 22 pivots in the direction of arrow C2a as illustrated in Fig. 6.

In the case where the first gear 61B rotates, similarly to the case where the first gear 61A rotates, the second and third gears 62B and 63B rotate with the rotating first gear 61B, and the first rod 132a, which meshes with the second gear 62B, and the second rod 132b, which meshes with the third gear 63B, move in opposing directions along the Z axis. The jaw 21 thereby pivots in the direction of arrow C2a or C2b as illustrated in Fig. 6.

In the case where the first gear 61C rotates, similarly to the cases where the first and second gears 61A and 61B rotate, the second and third gears 62C and 63C rotate with the rotating first gear 61C, and the first rod 133a, which meshes with the second gear 62C, and the second rod 133b, which meshes with the third gear 63C, move in opposing directions along the Z axis. The wrist section 23 thereby pivots in the direction of arrow B1 or B2 as illustrated in Fig. 3.

The driving mechanism 15 is not limited to the configuration including the three drive units 101. For example, when two pairs of two rods that move in opposing directions along the Z axis as illustrated in Fig. 7, the driving mechanism 15 includes two drive units 101 corresponding to the two pairs of rods.

When the driving mechanism 15 includes the three drive units 101, three members of the distal end part 11, including the jaw 21, jaw 22, and wrist section 23, are driven independently. This allows for fine manipulation during surgery. The driving mechanism 15 therefore preferably includes the three drive units 101.

Next, a description is given of other configuration examples of the invention with reference to the drawings. The same or equivalent portions in the drawings are given the same reference numerals, and the description thereof is not repeated.

In the description of the above configuration example, the shaft 12, which is provided for the medical treatment tool 4b, is a single unit connecting the distal end part 11 and driving mechanism 15.

In that case, the distal end part 11 cannot be detached from the shaft 12. In order to replace the distal end part 11, it is therefore necessary to change the entire medical treatment tool 4b. However, the structure to connect the medical treatment tool 4b and an arm section 10 is comparatively complicated, thus the work to replace the medical treatment tool 4b is not easy.

On the other hand, a medical treatment tool 204 according to this configuration example is configured so that members of a distal end part 211 can be detached from a shaft 212 and attached to the same. The distal end part 211 can thereby be replaced without changing the entire medical treatment tool 204. Hereinafter, the configuration of the medical treatment tool 204 according to this configuration example is described.

### (Configuration of Medical treatment tool (Example 1))

### (Entire Configuration)

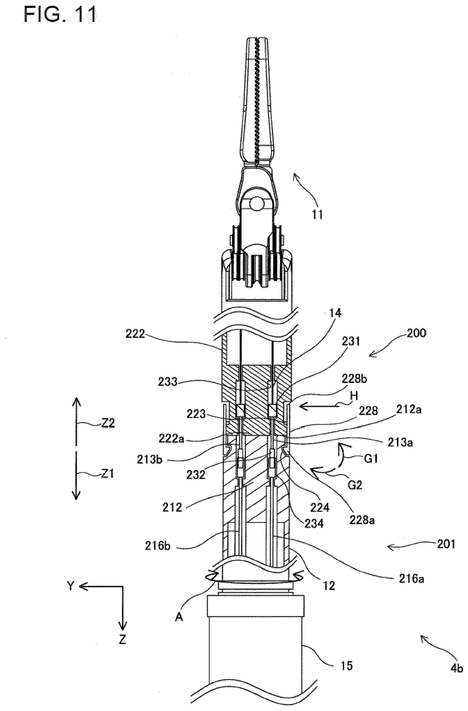

Fig. 11 is a cross-sectional view illustrating the configuration of a medical treatment tool (Example 1).

With reference to Fig. 11, the medical treatment tool 204 includes a treatment tool unit 200, the shaft 212, a driving mechanism 215, and plural driving-side rods (driving-side elongated elements) 216.

The driving mechanism 215 has the same configuration as the aforementioned driving mechanism 15. The driving mechanism 215 may have a different configuration from the driving mechanism 15 as long as the driving mechanism 215 is able to move the driving-side rods 216 in the longitudinal direction of the medical treatment tool 204.

The driving-side rods 216 are rigid members accommodated in the shaft 212. The driving-side rods 216 include a first driving-side rod (a first elongated element) 216a and a second driving-side rod (a second elongated element) 216b.

The medical treatment tool 204 includes three pairs of the first driving-side rod 216a and second driving-side rod 216b, for example. Fig. 11 illustrates the driving-side first and second rods 216a and 216b of one of the three pairs.

The treatment tool unit 200 is configured to be attachable to an end section 212a of the shaft 212 on the opposite side to the driving mechanism 215. With such a configuration, the type of instrument is changed by detaching one treatment tool unit 200 from the shaft 212 and then attaching another treatment tool unit 200 to the shaft 212 without changing the entire medical treatment tool 204. This is economical because of no need to prepare plural entire medical treatment tools 204. In addition, there is also no need to prepare plural medical treatment tools 204 for replacement during surgery, thereby leading to provision of sufficient work space for surgery.

To be more specific, the treatment tool unit 200 includes the distal end part 211, plural distal-side rods (rigid distal-side elongated elements) 213, a wire (a flexible elongated element) 214, an engaging section 221, and a cylindrical section 222.

As described above, the distal end part 211 has the same configuration as the distal end part 11 according to the aforementioned configuration example. The distal end part 211 moves through the wire 214 being driven, since the wire 214 is fixed thereto.

In such a manner, the configuration in which the distal end part 211 moves through the wire 214 being driven allows the distal end part 211 to move smoothly in a desired direction.

The distal-side rods 213 are rigid members accommodated in the cylindrical section 222 and includes a first distal-side rod (a third elongated element) 213a and a second distal-side rod (a fourth elongated element) 213b. The first and second distal-side rods 213a and 213b connect to the wire 214. The wire 214 includes sufficient strength, flexibility, and durability.

The treatment tool unit 200 includes three sets of the first distal-side rod 213a, second distal-side rod 213b, and wire 214, for example. Fig. 11 illustrates the first distal-side rod 213a, second distal-side rod 213b, and wire 214 of one of the three sets.

The end section (opening) 222a of the cylindrical section 222, which is located on the side of the driving mechanism 215 when the treatment tool unit 200 is attached to the shaft 212 (hereinafter, just referred to as the driving mechanism 215 side), has the substantially same diameter as that of the end section 212a of the shaft 212.

The engaging section 221 is substantially bar-shaped and is attached to near the end section 222a in the cylindrical section 222 through a joint section 223. The engaging section 221 protrudes from the end section 222a to the driving mechanism 215 side. The engaging section 221 is pivotable about the joint section 223 in the direction of arrow G1 or G2.

The first end 221a of the engaging section 221 on the driving mechanism 215 side protrudes toward the central axis of the shaft 212. The engaging section 221 is energized by a not-illustrated spring, for example so that the first end 221a pivots in the direction of arrow G2, that is, toward the center axis of the shaft 212.

The shaft 212 includes a cylindrical shape extended in the longitudinal direction of the medical treatment tool 204 in a similar manner to the shaft 12 according to the aforementioned configuration example. The shaft 212 is rotatable in the direction of arrow A. The shaft 212 accommodates the plural driving-side rods 216. In the end section 212a of the shaft 212, a recess 224, which is engageable with the first end 221a of the engaging section 221 in the treatment tool unit 200, is formed.

### (Attachment of Instrument Unit to Shaft)

To attach the treatment tool unit 200 to the shaft 212, an operator presses a second end 221b of the engaging section 221 in the direction of arrow H. The first end 221a of the engaging section 221 pivots about the joint section 223 in the direction of arrow G1.

In the state where the first end 221a is pivoted in the direction of arrow G1, the operator aligns the end section 222a of the cylindrical section 222 with the end section 212a of the shaft 212 and releases the pressure on the second end 221b in the direction of arrow H. The first end 221a then pivots about the joint section 223 in the direction of arrow G2 to engage with the recess 224. The treatment tool unit 200 is thus attached to the shaft 212.

To detach the treatment tool unit 200 from the shaft 212, the operator presses the second end 221b in the direction of arrow H again to pivot the first end 221a in the direction of arrow G1. The first end 221a is thereby disengaged from the recess 224. The operator moves the treatment tool unit 200 away from the shaft 212, so that the treatment tool unit 200 is detached from the shaft 212.

### (Engagement of Distal-side Rod and Driving-side Rod)

The plural distal-side rods 213 are engageable with the respective driving-side rods 216.

To be more specific, each of the distal-side rods 213 includes a thick section (a first end) 231, which is an end on the distal end part 211 side, and a pin (a second end) 232, which is an end on the driving mechanism 215 side. The driving-side rod 216 includes a connector 234, which is an end on the distal end part 211 side and accommodates the pin 232.

In the cylindrical section 222, a passage section 233 is formed. The passage section 233 accommodates the thick section 231 inside and limits movement of the thick section 231 along the Z-axis. The passage section 233 is formed so that the distal-side rod 213 and wire 214 can be inserted therethrough. Both ends of the passage section 233 have diameters small enough to prohibit the thick section 231 from passing through the same.

As described above, when the operator brings the treatment tool unit 200 close to the shaft 212 while pressing the second end 221b of the engaging section 221 in the direction of arrow H to pivot the first end 221a in the direction of arrow G1, the pin 232 comes into contact with the connector 234 and receives force from the connector 234 in the direction of arrow Z2.

The distal-side rod 213 moves in the direction of arrow Z2, and the thick section 231 comes into contact with the end of the passage section 233 on the distal end part 211 side. This limits movement of the distal-side rod 213 in the direction of arrow Z2. In this state, when the operator further moves the treatment tool unit 200 close to the shaft 212, the pin 232 is accommodated in the connector 234, so that the distal-side rod 213 and driving-side rod 216 engage with one another.

In the case where the operator detaches the treatment tool unit 200 from the shaft 212, the thick section 231 and pin 232 of the distal-side rod 213 and the passage section 233 of the cylindrical section 222 function as a releasing mechanism to disengage the distal-side rods 213 from the driving-side rods 216.

Specifically, when the operator moves the treatment tool unit 200 away from the shaft 212 while pressing the first end 221a in the direction of arrow H, the pin 232, which is in the connector 234, is pulled in the direction of arrow Z1.

The distal-side rod 213 thereby moves in the direction of arrow Z1. The thick section 231 of the distal-side rod 213 comes into contact with the end of the passage section 233 on the driving mechanism 151 side. This limits the movement of the distal-side rod 213 in the direction of arrow Z1. In this state, the operator further moves the treatment tool unit 200 away from the shaft 212. The pin 232 is then separated from the connector 234, so that the distal-side rod 213 is disengaged from the driving-side rod 216.

That configuration in which the end of the distal-side rod 213 on the driving mechanism 215 side is the pin 232 while the end of the driving-side rod 216 on the distal end part 211 side is the connector 234 allows for easy engagement and disengagement of the distal-side rod 213 and driving-side rod 216.

The distal end part 211 is not limited to the configuration in which the distal end part 211 is integrated with the treatment tool unit 200 and may be configured as a different body from the treatment tool unit 200.

The diameter of the end section 222a of the cylindrical section 222 may be larger than the diameter of the other part of the cylindrical section 222, that is, part thereof other than the end section 222a. Such a configuration prevents the components for engagement of the distal-side rod 213 and driving-side rod 216, including the pin 232 and connector 234, from being excessively miniaturized, thus facilitating manufacturing the components and ensuring strength of engagement.

The mechanism to engage the distal-side rod 213 and driving-side rod 216 is not limited to the configuration including the pin 232 and connector 234 as long as the mechanism includes sufficient strength and durability.

The end of the distal-side rod 213 on the driving mechanism 215 side may be made of an elastic member. In such an end of LAN (Local Area Network) cable, when pressurized in a predetermined direction, the end of the distal-side rod 213 on the driving mechanism 215 side deforms while being accommodated in a connector which is the end of the driving-side rod 216 on the distal end part 211 side. In this case, when the position of the distal-side rod 213 relative to the driving-side rod 216 reaches the predetermined position, the end of the distal-side rod 213 accommodated in the connector returns to the initial form within the connector and engages with a recess formed in the connector, for example.

The treatment tool unit 200 may be configured so as to separate into plural members. For example, the cylindrical section 222 is configured so as to separate into a member on the distal end part 211 side and a member on the driving mechanism 215 side, and the distal-side rod 213, which is accommodated in the cylindrical section 222, is also configured so as to separate into a member on the distal end part 211 side and a member on the driving mechanism 215 side. The treatment tool unit 200 thereby separates into two members.

The distal end part 211 is not limited to the configuration in which the distal end part 211 connects to the wire 214 and may directly connect to the distal-side rod 213. In such a configuration, no work is necessary to adjust the tension of the wire 214 during the assembly process of the medical treatment tool 204 and the like. It is therefore possible to further facilitate assembly of the medical treatment tool 204 and the like.

### (Configuration of Medical treatment tool (Example 2))

### (Attachment of Treatment tool unit to Shaft)

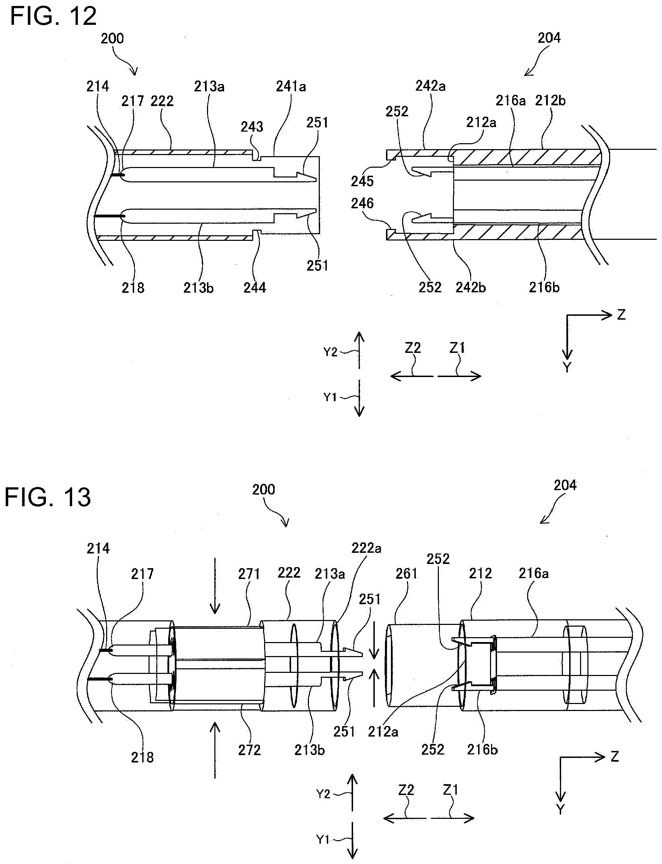

Fig. 12 is a cross-sectional view illustrating the configuration of a Medical treatment tool (Example 2). In Fig. 12, arrow Y1 indicates the positive direction of the Y axis while arrow Y2 indicates the negative direction of the Y axis.

With reference to Fig. 12, the cylindrical section 222 of the treatment tool unit 200 includes two first extensions 241a and 241b, which extend toward a driving mechanism 215. The two first extensions 241a and 241b are arranged next to each other in the direction perpendicular to the Y-Z plane. Fig. 12 illustrates only the first extension 241a and does not illustrate the first extension 241b.

The shaft 212 includes two second extensions 242a and 242b, which extend from the end section 212a to the distal end part 211 side. The two second extensions 242a and 242b are arranged next to each other in the direction parallel to the Y-Z plane.

The first extensions 241a and 241b of the treatment tool unit 200 connect to the second extensions 242a and 242b of the shaft 212 to form the outer circumference of the shaft 212.

Each of the first extensions 241a and 241b includes: a recess 243 formed in a part of the connection with the second extension 242a; and a recess 244 formed in a part of the connection with the second extension 242b. The second extension 242a includes a protrusion 245, which is engageable with the recess 243, and the second extension 242b includes a protrusion 246, which is engageable with the recess 244.

To attach the treatment tool unit 200 to the shaft 212, the operator moves the treatment tool unit 200 in the direction perpendicular to the Y-Z plane so that the protrusion 245 engages with the recess 243 while the protrusion 246 engages with the recess 244. The treatment tool unit 200 is thus attached to the shaft 212.

To detach the treatment tool unit 200 from the shaft 212, the operator moves the treatment tool unit 200 in the direction perpendicular to the Y-Z plane. The protrusions 245 and 246 are disengaged from the recesses 243 and 244, respectively, so that the treatment tool unit 200 is detached from the shaft 212.

### (Engagement of Distal-side Rod and Driving-side Rod)

The treatment tool unit 200 includes three sets of the first distal-side rod 213a, second distal-side rod 213b, and wire 214. The three first distal-side rods 213a included in the respective three sets are arranged next to each other in the direction perpendicular to the Y-Z plane. The three second distal-side rods 213b included in the respective three sets are also arranged next to each other in the direction perpendicular to the Y-Z plane.

The first and second distal-side rods 213a and 213b connect to the wire 214. An end section 217 of the first distal-side rod 213a on the wire 214 side and an end section 218 of the second distal-side rod 213b on the wire 214 side are both tapered.

In such a configuration, even when the driven wire 214 comes into contact with the end section of the distal-side rod 213 connecting to another wire 214, the wire 214 is prevented from being damaged by the angular end of the another wire 214.

Each of the first and second distal-side rods 213a and 213b includes a first engagement section 251, which is provided at an end thereof on the driving mechanism 215 side. The first engagement section 251 of the first distal-side rod 213a protrudes in the direction of arrow Y2, for example. The first engagement section 251 of the second distal-side rod 213b protrudes in the direction of arrow Y1, for example.

The medical treatment tool 204 includes three sets of the driving-side first and second rods 216a and 216b. The three first driving-side rods 216a included in the respective three sets are arranged next to each other in the direction perpendicular to the Y-Z plane. The three second driving-side rods 216b included in the respective three sets are also arranged next to each other in the direction perpendicular to the Y-Z plane.

Each of the driving-side first and second rods 216a and 216b includes a second engagement section 252, which is provided at an end on the distal end part 211 side when the treatment tool unit 200 is attached to the shaft 212 (hereinafter, just referred to as a distal end part 211 side). The second engagement section 252 of the first driving-side rod 216a protrudes in the direction of arrow Y1, for example. The second engagement section 252 of the second driving-side rod 216b protrudes in the direction of arrow Y2, for example.

As described above, when the operator moves the treatment tool unit 200 in the direction perpendicular to the Y-Z plane to attach the treatment tool unit 200 to the shaft 212, the second engagement sections 252 engage with the respective first engagement sections 251. The distal-side rods 213 and respective driving-side rods 216 thus engage with one another.

In the process where the operator detaches the treatment tool unit 200 from the shaft 212, the first and second engagement sections 251 and 252 function as a releasing mechanism to disengage the distal-side rods 213 from the driving-side rods 216.

Specifically, when the operator moves the treatment tool unit 200 in the direction perpendicular to the Y-Z plane to detach the treatment tool unit 200 from the shaft 212, the first engagement sections 251 are disengaged from the second engagement sections 252, so that the distal-side rods 213 are thereby disengaged from the respective driving-side rods 216.

The treatment tool unit 200 unnecessarily includes the wire 214. In such a case, the distal end part 211 directly connects to the first and second distal-side rods 213a and 213b and moves through the first and second distal-side rods 213a and 213b being driven.

### (Configuration of Medical treatment tool (Example 3))

### (Attachment of Treatment tool unit to Shaft)

Fig. 13 is a perspective view illustrating the configuration of a medical treatment tool (Example 3).

With reference to Fig. 13, the shaft 212 includes a fitting section 261, which extends from the end section 212a toward the distal end part 211. The fitting section 261 is cylindrical, and the outer diameter thereof is slightly smaller than the inner diameter of the end section 222a of the cylindrical section 222 of the treatment tool unit 200. The treatment tool unit 200 is thereby attached to the shaft 212 by bringing the treatment tool unit 200 close to the shaft 212 and fitting the fitting section 261 into the end section 222a.

To detach the treatment tool unit 200 from the shaft 212, the operator moves the treatment tool unit 200 away from the shaft 212 to detach the treatment tool unit 200 from the shaft 212.

### (Engagement of Distal-side Rod and Driving-side Rod)

The treatment tool unit 200 includes three sets of the first distal-side rod 213a, second distal-side rod 213b, and wire 214 in a similar manner to Example 2 described above. The three first distal-side rods 213a included in the respective three sets are arranged next to each other in the direction perpendicular to the Y-Z plane. The three second distal-side rods 213b included in the respective three sets are also arranged next to each other in the direction perpendicular to the Y-Z plane.

The first and second distal-side rods 213a and 213b connect to the wire 214. The end section 217 of the first distal-side rod 213a on the wire 214 side and the end section 218 of the second distal-side rod 213b on the wire 214 side are both tapered.

Each of the first and second distal-side rods 213a and 213b includes the first engagement section 251, which is provided at the end thereof on the driving mechanism 215 side. The first engagement section 251 of the first distal-side rod 213a protrudes in the direction of arrow Y2, for example. The first engagement section 251 of the second distal-side rod 213b protrudes in the direction of arrow Y1, for example.

The three first distal-side rods 213a are fixed to a pressing section 271, which constitutes a part of the outer circumference of the cylindrical section 222. The pressing section 271 is energized in the direction of arrow Y2 by a not-illustrated spring, for example.

The three second distal-side rods 213b are fixed to a pressing section 272, which constitutes a part of the outer circumference of the cylindrical section 222. The pressing section 272 is energized in the direction of arrow Y1 by a not-illustrated spring, for example.

The medical treatment tool 204 includes three sets of the first and second driving-side rods 216a and 216b. The three first driving-side rods 216a included in the respective three sets are arranged next to each other in the direction perpendicular to the Y-Z plane. The three second driving-side rods 216b included in the respective three sets are also arranged next to each other in the direction perpendicular to the Y-Z plane.

Each of the first and second driving-side rods 216a and 216b includes a second engagement section 252, which is provided at an end on the distal end part 211 side. The second engagement section 252 of the first driving-side rod 216a protrudes in the direction of arrow Y1, for example. The second engagement section 252 of the second driving-side rod 216b protrudes in the direction of arrow Y2, for example.

To attach the treatment tool unit 200 to the shaft 212, the operator just needs to simply insert the cylindrical section 222 into the fitting section 261. The first engagement section 251 of the first distal-side rods 213a is tapered. As the cylindrical section 222 moves in the direction of arrow Z1, therefore, the two first engagement sections 251 properly move close to each other along the Y axis and finally engage with the second engagement sections 252.

To attach the treatment tool unit 200 to the shaft 212 more reliably, the operator presses the pressing section 271 in the direction of arrow Y1 and presses the pressing section 272 in the direction of arrow Y2. The first engagement section 251 of the first distal-side rod 213a thereby moves in the direction of arrow Y1 while the first engagement section 251 of the second distal-side rod 213b moves in the direction of arrow Y2.

In this state, the operator brings the treatment tool unit 200 close to the shaft 212 so that the first engagement sections 251 overlap the second engagement sections 252 on the Y-axis. The operator then stops pressing the pressing sections 271 and 272, the first engagement sections 251 engage with the respective second engagement sections 252. The distal-side rods 213 thus engage with the respective driving-side rods 216.

In the case where the operator detaches the treatment tool unit 200 from the shaft 212, the first engagement sections 251, second engagement sections 252, and pressing sections 271 and 272 function as a releasing mechanism to disengage the distal-side rods 213 from the driving-side rods 216.

Specifically, when the operator presses the pressing section 271 in the direction of arrow Y1 and presses the pressing section 272 in the direction of arrow Y2 to detach the treatment tool unit 200 from the shaft 212, the first engagement sections 251 are disengaged from the second engagement sections 252. When the operator moves the treatment tool unit 200 away from the shaft 212 in this state, the distal-side rods 213 are disengaged from the driving-side rods 216.

The treatment tool unit 200 unnecessarily includes the wire 214. In this case, the distal end part 211 directly connects to the first and second distal-side rods 213a and 213b and moves through the first and second distal-side rods 213a and 213b being driven.

The other configurations and movements are the same as those of the aforementioned configuration examples, and the detailed description thereof is not repeated.

The characteristics of the aforementioned treatment tool unit 200 and the medical treatment tool 204 and surgical system 300 including the same are summarized as follows.
[1] A treatment tool unit, which is attachable to a shaft that accommodates rigid driving-side elongated elements to operate the distal end part of a medical treatment tool, the treatment tool unit including:
   a cylindrical section including an opening having the substantially same diameter as that of an end of the shaft on the tip-section side; and
   rigid distal-side elongated elements accommodated in the cylindrical section, in which the distal-side elongated elements include a first end located on the tip-section side and a second end which is engageable with an end of the corresponding one of the driving-side elongated elements on the driving-section side.
[2] The treatment tool unit according to [1], in which the second end is a pin, and
   the end of the driving-side elongated element on the tip-section side is a connector to accommodate the pin.
[3] The treatment tool unit according to [1] or [2] in which the distal-side elongated element includes a thick section, and the cylindrical section includes a passage section that limits a range of movement of the thick section.
[4] The treatment tool unit according to any one of [1] to [3], the cylindrical section includes an engagement section that is engageable with the shaft
[5] The treatment tool unit according to any one of [1] to [4)] further including a releasing mechanism to disengage the driving-side elongated element from the distal-side elongated element.
[6] The treatment tool unit according to [5], in which the releasing mechanism is provided for the cylindrical section, and the releasing mechanism is pressed toward the inside of the cylindrical section to release the engagement.
[7] The treatment tool unit according to any one of [1] to [6], further including the distal end part that is operated directly or indirectly by the distal-side elongated element being driven.
[8] The treatment tool unit according to [7], in which the distal-side elongated element connects to a flexible elongated element, and
   the distal end part is operated through movement of the flexible elongated element.
[9] The treatment tool unit according to [8], in which an end of the distal-side elongated element on the flexible elongated element's side is tapered.
[10] The treatment tool unit according to any one of [1] to [9], in which the diameter of the opening is greater than the diameter of the other part of the cylindrical section than the opening.
[11] A medical treatment tool, including: the treatment tool unit according to any one of [7] to [10]; the distal end part; the shaft; and a drive unit to drive the distal end part.
[12] The medical treatment tool according to [11], in which the drive unit includes:
   a first gear that rotates about a first axis;
   a second gear that rotates about a second axis extending in a direction perpendicular to the first axis; and
   a third gear that rotates about a third axis extending in the direction perpendicular to the first axis, and
   the driving-side elongated elements include:
      a first elongated element that meshes with the second gear; and
      a second elongated element that meshes with the third gear, and
      the second and third gears mesh with the first gear, and
      the distal end part is operated in such a manner that as the first gear rotates, the first elongated element moves in a longitudinal direction of the medical treatment tool while the second elongated element moves in a direction opposite to the direction in which the first elongated element moves.
[13] The medical treatment tool according to [12], in which the first, second, and third gears are bevel gears.
[14] The medical treatment tool according to [12] or [13], in which the first gear connects to a receiver member which is engageable with a transmitter member that is driven by an actuator.
[15] The medical treatment tool according to any one of [11] to [14], further comprising a driving mechanism including three of the drive units.
[16] A surgical system, including: the medical treatment tool according to [14] or [15] referring to [14]; and a manipulator including the transmitter member that engages with the receiver member.

The above-described embodiments should be considered to be illustrative in all respects and not restrictive. The scope of the invention is indicated by the appended claims, rather than by foregoing description, and is intended to include all alterations within the meaning and range equivalent to the claims.

### EXPLANATION OF REFERENCES

3 MANIPULATOR
4b MEDICAL TREATMENT TOOL
11 DISTAL END PART
12 SHAFT
13 ROD (RIGID ELONGATED ELEMENT)
13a, 131a, 132a, 133a FIRST ROD (FIRST RIGID ELONGATED ELEMENT)
13b, 131b, 132b, 133b SECOND ROD (SECOND RIGID ELONGATED ELEMENT)
14, 141, 142, 143 WIRE (FLEXIBLE ELONGATED ELEMENT)
15 DRIVING MECHANISM
50, 51, 52, 53, 54 ACTUATOR (ROTATION MECHANISM)
61, 61A, 61B, 61C FIRST GEAR
62, 62A, 62B, 62C SECOND GEAR
63, 63A, 63B, 63C THIRD GEAR
77 RECEIVER MEMBER
78 TRANSMITTER MEMBER
101 DRIVE UNIT
161 FIRST AXIS
162 SECOND AXIS
163 THIRD AXIS
200 TREATMENT TOOL UNIT
300 SURGICAL SYSTEM

## Claims

1. A drive unit for driving elongated elements to operate a distal end part of a medical treatment tool, the drive unit comprising:
a first gear that rotates about a first axis;
a second gear that rotates about a second axis extending in a direction perpendicular to the first axis; and
a third gear that rotates about a third axis extending in a direction perpendicular to the first axis, wherein
the elongated elements include:
a first elongated element that is rigid and meshes with the second gear; and
a second elongated element that is rigid and meshes with the third gear,
the second and third gears mesh with the first gear, and
the distal end part is operated in such a manner that as the first gear rotates, the first elongated element moves in a longitudinal direction of the medical treatment tool while the second elongated element moves in a direction opposite to the direction in which the first elongated element moves.

2. The drive unit according to claim 1, wherein
a position where the first elongated element is engaged with a side surface of a disk of the second gear as seen from the first gear is opposite to a position where the second elongated element is engaged with a side surface of a disk of the third gear as seen from the first gear.

3. The drive unit according to claim 1 or 2, wherein
the first, second, and third gears are bevel gears.

4. The drive unit according to claim 1 or 2, wherein
the first gear is a crown gear, and the second and third gears are spur gears.

5. The drive unit according to any one of claims 1 to 4, wherein
the first and second elongated elements individually connect to a flexible elongated element, and
the distal end part is operated in response to movements of the flexible elongated element.

6. The drive unit according to any one of claims 1 to 5, wherein
the first gear connects to a receiver member which is engageable with a transmitter member that is driven by an actuator.

7. A medical treatment tool, comprising:
the drive unit according to any one of claims 1 to 6;
the distal end part that is operated by the first and second elongated elements; and
a shaft that accommodates the first and second elongated elements.

8. The medical treatment tool according to claim 7, wherein
the distal end part is detachable from the shaft.

9. The medical treatment tool according to claim 7 or 8, further comprising:
a third elongated element that is rigid and is engageable with the first elongated element; and
a fourth elongated element that is rigid and is engageable with the second elongated element.

10. A medical treatment tool, comprising:
a driving mechanism that includes three of the drive units according to any one of claims 1 to 6;
the distal end part that is operated with three pairs of the first and second elongated elements; and
a shaft that accommodates the three pairs of the first and second elongated elements.

11. The medical treatment tool according to claim 10, wherein
the first axes of the three drive units are positioned at equal intervals.

12. A surgical system, comprising:
the medical treatment tool according to any one of claims 7 to 11 referring to claim 6; and
a manipulator including the transmitter member that engages with the receiver member.

13. The surgical system according to claim 12, wherein
the manipulator includes a rotation mechanism that rotates the shaft.

14. The surgical system according to claim 13, wherein
the rotation mechanism includes:
a rotational axis adjustment gear fixed such that a central axis of the rotational axis adjustment gear coincides with a rotational axis of the shaft; and
a gear that rotates about an axis thereof while revolving around the rotational axis adjustment gear.

15. The surgical system according to claim 13 or 14, wherein
the manipulator further includes:
three of the actuators that drive three of the transmitter members; and
a rotation mechanism driving actuator that drives the rotation mechanism.
